# EUROPÄISCHE PATENTSCHRIFT

(11) **EP 4 076 574 B1**
(45) Veröffentlichungstag und Bekanntmachung des Hinweises auf die Patenterteilung: **04.06.2025**
(21) Anmeldenummer: 20838919.7
(22) Anmeldetag: 15.12.2020
(51) Int. Cl.: A61M 1/14, A61M 1/16, A61M 1/36

(54) **SET MIT EFFLUENTBEUTEL UND ADAPTER**
SET COMPRISING EFFLUENT BAG AND ADAPTER
ENSEMBLE COMPRENANT UN SAC POUR L'EFFLUENT ET UN ADAPTEUR

(30) Priorität: 19.12.2019 DE 102019135229
(43) Veröffentlichungstag der Anmeldung: 26.10.2022
(73) Patentinhaber: Fresenius Medical Care Deutschland GmbH, 61352 Bad Homburg (DE)
(72) Erfinder: KLEWINGHAUS, Juergen, 61440 Oberursel (DE)
(74) Vertreter: Bobbert & Partner Patentanwälte PartmbB
(86) Internationale Anmeldenummer: PCT/EP2020/086192
(87) Internationale Veröffentlichungsnummer: WO 2021/122577

(56) Entgegenhaltungen:
- WO-A1-2019/016145

## Beschreibung

Die vorliegende Erfindung betrifft ein Set mit einem Effluentbeutel gemäß Anspruch 1 und ein Verfahren gemäß Anspruch 10 zum Entleeren eines Effluentbeutels.

Aus der Praxis ist die extrakorporale Blutbehandlung bekannt. Dabei wird dem Patienten Blut entnommen, das entlang eines Blutkreislaufs extrakorporal und z. B. durch einen Blutfilter geführt wird. Der Blutfilter weist eine Blutkammer, durch welche Blut geführt wird, und eine Dialysierflüssigkeitskammer, durch welche Dialysierflüssigkeit geführt wird, auf. Beide Kammern sind durch eine semi-permeable Membran voneinander getrennt. Blut und Dialysierflüssigkeit werden zumeist im Gegenstromprinzip durch den Blutfilter geleitet. Das Blut wird im Blutfilter gereinigt, die Dialysierflüssigkeit gilt bei ihrem Austritt aus dem Blutfilter als verbraucht und wird, nun als Effluent bezeichnet, verworfen. Neben dem Dialysat umfasst das zu verwerfende Effluent auch Filtrat (oder Ultrafiltrat), welches Wasser, das dem Blut im Blutfilter entzogen wurde, umfasst. Filtrat und Dialysat werden im Folgenden einzeln oder gemeinsam vereinfacht als Effluent bezeichnet.

Das Effluent wird in der Praxis mittels einer Effluentzulaufleitung einem Effluentbeutel zugeführt und darin zunächst aufbewahrt. Insbesondere nach Beendigung der Blutbehandlung wird das Effluent aus dem Effluentbeutel, der über ein Waschbecken oder einen Ausguss gehalten wird, in diesen hinein verworfen.

WO 2019/016145 A1 offenbart einen Effluentbeutel zum Aufnehmen des bei einer Blutbehandlung anfallenden Effluents. Eine Aufgabe der vorliegenden Erfindung kann darin bestehen, ein Set mit einem Effluentbeutel zum Einsatz bei der Blutbehandlung anzugeben.

Ferner soll ein Verfahren zum Entleeren des Effluentbeutels angegeben werden.

Die erfindungsgemäße Aufgabe kann durch ein Set mit einem Effluentbeutel mit den Merkmalen des Anspruchs 1 gelöst werden. Zudem kann sie durch ein Verfahren mit den Merkmalen des Anspruchs 10 gelöst werden.

Alle mit dem erfindungsgemäßen Verfahren erzielbaren Vorteile lassen sich in bestimmten erfindungsgemäßen Ausführungsformen ungeschmälert auch mit dem erfindungsgemäßen Set erzielen.

Die vorliegende Erfindung betrifft ein Set mit einem Effluentbeutel, welcher ausgestaltet ist, um bei einer Blutbehandlung anfallendes Effluent aufzunehmen. Der erfindungsgemäße Effluentbeutel umfasst wenigstens eine oder genau eine, optional verschließbare, Effluentöffnung zu einem Äußeren des Effluentbeutels. Die Effluentöffnung weist einen ersten Konnektor von einem ersten Typ auf oder ist hiermit verbunden.

Ferner umfasst das Set einen Adapter mit einem Leitungsabschnitt zum Leiten eines Fluids, z. B. Effluent, wobei der Leitungsabschnitt einen zweiten Konnektor vom ersten Typ und einen dritten Konnektor von einem zweiten Typ aufweist. Der erste und der zweite Typ sind verschieden voneinander.

Die vorliegende Erfindung betrifft weiter ein Verfahren zum Entleeren eines Effluentbeutels. Das Verfahren umfasst ein Bereitstellen eines erfindungsgemäßen Sets, wobei der erste Konnektor (vom ersten Typ) des Effluentbeutels, zum Aufnehmen des bei einer Blutbehandlung angefallenen Effluents im Effluentbeutel, mit dem zweiten Konnektor (vom ersten Typ) des Adapters verbunden ist. Außerdem ist der dritte Konnektor (vom zweiten Typ) des Adapters mit einem fünften Konnektor (vom zweiten Typ) der Effluentzulaufleitung der Blutbehandlungsvorrichtung verbunden. Das erfindungsgemäße Verfahren umfasst weiter ein Trennen des ersten Konnektors (vom ersten Typ) vom zweiten Konnektor (vom ersten Typ) und ein erneutes Verbinden des ersten Konnektors (vom ersten Typ), und zwar mit dem vierten Konnektor (vom ersten Typ) der Effluentablaufleitung. Dies erfolgt derart, dass eine Fluidverbindung zwischen dem Inneren des Effluentbeutels und dem Inneren der Effluentablaufleitung hergestellt wird. Diese Fluidverbindung dient zum Abführen von Effluent aus dem Effluentbeutel bzw. zu dessen Entleeren.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche. Die Ansprüche definieren den Schutzbereich.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Wenn hierin von einer Ausführungsform die Rede ist, so stellt diese eine erfindungsgemäße, beispielhafte Ausführungsform dar.

In einigen Ausführungsformen ist der Leitungsabschnitt des Adapters eine nicht biegsame Kunststoffleitung, in anderen ein, vorzugsweise biegsamer, Schlauchabschnitt, oder weist solche jeweils auf.

In manchen Ausführungsformen steht die Effluentöffnung des Effluentbeutels mit dem ersten Konnektor desselben, der vom ersten Typ ist, mittels eines, vorzugsweise biegsamen, Schlauchabschnitts in Fluidverbindung. Hierbei ist zwischen Effluentöffnung und erstem Konnektor vom ersten Typ vorzugsweise ein erster Innendurchmesser für den Schlauchabschnitt vorgesehen.

In einigen Ausführungsformen weist der Leitungsabschnitt des Adapters einen zweiten Innendurchmesser auf, welcher geringer ist als der erste Innendurchmesser.

In manchen Ausführungsformen sind der Adapter, oder der als Schlauchabschnitt ausgestaltete Leitungsabschnitt des Adapters, und/oder die Effluentzulaufleitung mit einer äußeren Oberfläche des Effluentbeutels, vorzugsweise lösbar, verbunden. Sie können daher vorteilhafterweise am Effluentbeutel verbleiben, bis sie (erneut) benötigt werden. Ebenso kann z. B. der Adapter nach Gebrauch wieder an einer optional vorgesehenen Halterung lösbar angeordnet werden.

Eine Halterung zum vorübergehenden Halten des Adapters und/oder der Effluentzulaufleitung kann nicht nur am Effluentbeutel vorgesehen sein wie vorstehend beschrieben, etwa in oder nahe einer Griffleiste oder nahe zu Befestigungsöffnungen des Effluentbeutels, sondern ergänzend oder alternativ auch an der Blutbehandlungsvorrichtung, z. B. als Weiterbildung ihrer Haken.

Die vorstehend genannte Halterung kann dem Halten des Adapters und/oder der Effluentzulaufleitung in einer Parkposition dienen, bis diese bestimmungsgemäß benötigt werden.

Die Halterung kann als Clip, Klemmverbindung, Durchstecklösung, usw. ausgestaltet sein.

Die Halterung ist vorzugsweise in einem oberen Bereich des Effluentbeutels (z. B. in dessen oberen Drittel) oder an einem Abschnitt der Blutbehandlungsvorrichtung vorgesehen, da der mittels der Halterung gehaltene Adapter und/oder die gehaltene Effluentzulaufleitung auf diese Weise weniger Bewegungsspielraum etwa zum Schwingen hat, was der Genauigkeit der mittels der Wiegevorrichtung gemessenen Werte zugutekommen kann.

In einigen Ausführungsformen des erfindungsgemäßen Sets weist dieses weiter eine Effluentablaufleitung zum Abführen von Effluent aus dem Effluentbeutel auf. Hierbei weist die Effluentablaufleitung an dem zu ihrem Verbinden bestimmungsgemäß vorgesehenen Ende oder an der entsprechenden Öffnung einen vierten Konnektor vom ersten Typ auf. Alternativ oder ergänzend weist sie einen Innendurchmesser auf, welcher dem ersten Innendurchmesser entspricht.

In manchen Ausführungsformen weist das erfindungsgemäße Set ferner wenigstens eine Pumpe oder einen Pumpkopf einer Pumpe auf. Die Pumpe oder der Pumpkopf ist mit der Effluentablaufleitung verbunden und dient zum Pumpen von Effluent aus dem Effluentbeutel heraus. Alternativ sind Pumpe oder Pumpenkopf zu ihrer Verbindung hierzu vorgesehen.

In einigen Ausführungsformen weist das Set weiter eine Blutbehandlungsvorrichtung, verbunden mit einer Effluentzulaufleitung, auf. Die Effluentzulaufleitung dient zum Zuführen von, bei einer Blutbehandlungssitzung angefallenem, Effluents in den Effluentbeutel hinein. Hierbei weist die Effluentzulaufleitung einen fünften Konnektor vom zweiten Typ auf.

In manchen Ausführungsformen ist die Blutbehandlungsvorrichtung als Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = *continuous renal replacement therapy*)*.*

In einigen Ausführungsformen des erfindungsgemäßen Verfahrens umfasst dieses das Fördern von Effluent aus dem Effluentbeutel heraus, beispielsweise mittels der Pumpe, nachdem der erste Konnektor (vom ersten Typ) des Effluentbeutels mit dem vierten Konnektor (vom ersten Typ) der Effluentablaufleitung verbunden wurde.

Das erfindungsgemäße Set kann optional als Kit oder Abflussschlauchsystem (oder Teil hiervon) verstanden werden, welches in manchen Ausführungsformen weitere Komponenten (wie eine Ladestation für die Pumpe usw.) aufweist, welche nicht zum Fördern von Fluiden mit dem geförderten Fluid in Kontakt stehen.

In manchen Ausführungsformen umfasst der Effluentbeutel genau eine Effluentöffnung, die sowohl als Effluenteinlassöffnung als auch als Effluentauslassöffnung dient. Sie kann die einzige Flüssigkeitsöffnung des Effluentbeutels sein, sie kann die einzige Öffnung überhaupt des Effluentbeutels sein.

In einigen Ausführungsformen weist der Effluentbeutel, z. B. zusätzlich zur vorstehend genannten einzigen Effluentöffnung, eine weitere Öffnung, insbesondere eine Entlüftungsöffnung auf, die bestimmungsgemäß jedoch nicht dem Befüllen des Effluentbeutels mit Effluent oder seinem Entleeren dient. Solche weiteren Öffnungen haben optional keine Konnektoren zu ihrer Verbindung mit Fluidleitungen, optional haben sie Konnektoren, die jedoch nicht vom ersten Typ und/oder nicht vom zweiten Typ sind.

In einigen Ausführungsformen ist der Effluentbeutel oder seine Effluentöffnung stets mit nur einem Schlauchabschnitt, Verbinder oder nur einer Leitung verbunden.

In einigen Ausführungsformen ist die Effluentöffnung verschließbar und/oder mit einem Deckel vorgesehen.

In einigen Ausführungsformen kann der Effluentbeutel ein Behälter jeder Art sein, beispielsweise ein Container mit flexibler Außenhaut wie einer Folie, oder aus Folie bestehen, ein Container mit einer harten Außenhaut, oder aus einer harten Außenhaut bestehen, wie ein Kanister, usw.

In einigen Ausführungsformen ist der Effluentbeutel mit einem Abflussschlauchsystem verbunden oder verbindbar angeordnet oder ist ein Teil hiervon. Das Abflussschlauchsystem, oder Teile hiervon, weisen wenigstens, oder nur, eine Leitung auf, nämlich die zum Effluentbeutel führende Effluentzulaufleitung. Die Effluentzulaufleitung kann Teil des Sets sein. Sie ist hierin als Zulaufleitung bezeichnet, weil sie, obwohl sie im Gebrauch Effluent als Dialysatablaufleitung von der Blutbehandlungsvorrichtung wegleitet, dieses Effluent dem Effluentbeutel zuführt.

Die Effluentzulaufleitung dient dem Befüllen des Effluentbeutels. Klemmen können vorgesehen sein, um ein unerwünschtes Austreten von Effluent aus der Blutbehandlungsvorrichtung, während die Effluentzulaufleitung während des Entleerens des Effluentbeutels nicht mit dem Effluentbeutel verbunden ist, zu verhindern.

Optional weist das Set eine vom Effluentbeutel wegführende Effluentablaufleitung, aber keine, vorzugsweise elektrisch isolierende, Umschalteinrichtung auf, mittels welcher eine alternative oder zusätzliche Fluidverbindung hergestellt werden könnte. Das Set weist in diesen Ausführungsformen somit etwa keinen Drei-Wege-Hahn (alternativ als Drei-WegeVentil bezeichnet), keine Umschalteinrichtung aus Glas oder Kunststoff, keine Umschalteinrichtung die zwischen genau zwei Stellungen umschaltbar wäre und/oder keine Umschalteinrichtung auf, die als ein Mehr-Wege-Hahn oder -Ventil mit drei, genau drei, und/oder mehr als drei Wegen oder Anschlüssen ausgestaltet ist.

In bestimmten Ausführungsformen steht die Effluentablaufleitung mit wenigstens einer Pumpe oder einem Pumpenantrieb einer Pumpe, welche z. B. mit dem Pumpkopf zum Fördern zusammenwirkt, in Förderverbindung.

In manchen Ausführungsformen weist diese Pumpe oder dieser Pumpenantrieb wenigstens einen magnetisch gelagerten oder angetriebenen Pumpabschnitt, insbesondere einen Pumpkopf, auf. Dieser Pumpabschnitt oder Pumpkopf ist beispielsweise ausgestaltet als Impellerpumpkopf oder als dessen Rotor.

In einigen Ausführungsformen wird der Pumpenantrieb, insbesondere einer Blutbehandlungsvorrichtung oder einer nicht zur Blutbehandlungsvorrichtung zählenden Pumpe, manuell mit dem Pumpkopf des Abflussschlauchsystems verbunden.

In manchen Ausführungsformen wird der Betrieb des Pumpenantriebs manuell begonnen und/oder beendet.

In einigen Ausführungsformen umfasst die Blutbehandlungsvorrichtung eine Ladestation für eine Spannungsquelle für den Pumpenantrieb der Pumpe. Die Spannungsquelle kann eine Niedervolt- oder Niederstrom-Quelle sein.

In einigen Ausführungsformen umfasst das erfindungsgemäße Set die Ladestation für eine Spannungsquelle für den Pumpenantrieb der Pumpe, beispielsweise für einen Akku.

In manchen Ausführungsformen weist das Set wenigstens ein Rückschlagventil, z. B. stromab der optionalen Pumpe, vorzugweise in der Effluentablaufleitung, auf. Das Rückschlagventil kann ein ungewolltes Austreten von Effluent aus der Effluentablaufleitung vorteilhaft verhindern, was der Sauberkeit und Hygiene zugutekommt.

In einigen Ausführungsformen kann z. B. die Pumpe automatisch starten, wenn - manuell oder automatisch - die Effluentablaufleitung mit dem Effluentbeutel verbunden ist oder wird. Ergänzend oder alternativ kann z. B. die Pumpe automatisch stoppen, wenn - manuell oder automatisch - die Effluentzulaufleitung mit dem Effluentbeutel verbunden ist oder wird. Hierzu nötige Sensoren, Einrichtungen, usw. können vorgesehen sein.

Das Set kann disposable sein.

In manchen Ausführungsformen wird der Effluentbeutel zu seinem Entleeren oder für wenigstens einen Entleerungsvorgang nicht von seinem Platz, der Wiegeeinrichtung und/oder der Blutbehandlungsvorrichtung abgenommen oder entfernt.

In manchen Ausführungsformen wird das Effluent aus dem Effluentbeutel ohne Einsatz einer Pumpe entfernt, in anderen Ausführungsformen erfolgt dies unter Einsatz der Pumpe des Sets.

In einigen Ausführungsformen umfasst das Verfahren ein Schließen eines in der Effluentablaufleitung angeordneten Absperrelements, z. B. vorstehend genannter Klemme. Dies dient dem Absperren eines Fluidstroms über das Absperrelement hinweg.

In manchen Ausführungsformen wird das Absperrelement erst dann geöffnet, wenn die Pumpe für das Effluent, die in oder an der Effluentzulaufleitung angeordnet ist, angehalten wurde.

In einigen Ausführungsformen weisen Komponenten des erfindungsgemäßen Sets stromab des Effluentbeutels keinen Flussteiler auf.

Das Set kann hingegen eine Rollenpumpe aufweisen, etwa als in der Effluentablaufleitung angeordnete Pumpe. Diese Rollenpumpe kann optional mit genau einer Zuleitung und mit genau einer Ableitung verbunden sein. Damit kann die Pumpe den in sie hineinführenden Fluss offensichtlich nicht in mehrere Flüsse teilen.

In einigen Ausführungsformen weist das Set stromab der Pumpe der Effluentablaufleitung kein Element auf, das mit einer elektrischen Steuereinrichtung verbunden wäre, etwa in Form eines elektrisch verbundenen Verbinders.

In manchen Ausführungsformen weist die Effluentzulaufleitung kein Ventil, insbesondere kein Rückschlagventil, auf. Dasselbe kann ergänzend oder alternativ für jede(s) andere Komponente oder Bauteil des Sets gelten.

In einigen Ausführungsformen erfolgt das Befüllen und/oder das Entleeren des Effluentbeutels nicht mittels Steuerung oder Regelung einer Steuer- oder Regelvorrichtung.

Manche oder alle erfindungsgemäßen Ausführungsformen können einen, mehrere oder alle der oben und/oder im Folgenden genannten Vorteile aufweisen.

Ein Vorteil der vorliegenden Erfindung besteht darin, dass der erfindungsgemäße Effluentbeutel, anders als es bei herkömmlichen Auffangbeuteln der Fall ist, nicht manuell von der Maschine entfernt und über z. B. einem Ausgussbecken entleert werden muss, was, auch angesichts des Gewichts des Effluentbeutels von bis zu 10kg, eine unbeliebte, zeitaufwendige und körperlich anstrengende Tätigkeit darstellt. Er kann zu seinem Entleeren vielmehr an der Wiegeeinrichtung der Blutbehandlungsmaschine verbleiben.

Durch die vorliegende Erfindung wird vorteilhafterweise das Risiko umgangen, dass beim Ablassen des Inhalts des Effluentbeutels im Fehlerfall ein elektrisch leitender Kontakt von der Flüssigkeit zur Erde auftritt, so dass die zulässigen Patientenableitströme im Fehlerfall möglicherweise überschritten würden.

Eine Gefahr der elektrischen Erdung der Blutbehandlungsvorrichtung im Fehlerfall mit den bekannten Risiken für den Patienten lassen sich erfindungsgemäß wie folgt ausschließen: Der Effluentbeutel, dessen Inhalt bei seinem Entleeren mittels des Ausgusses, in welchen der Inhalt abgepumpt wird, geerdet werden könnte, steht während des Entleerens nicht in Kontakt oder Flüssigkeitskontakt mit der Blutbehandlungsvorrichtung, da er - aufgrund der Tatsache, dass er nur eine Öffnung für Effluent aufweisen muss - entweder mit der Blutbehandlungsvorrichtung oder mit dem Ausguss in Verbindung stehen kann, nicht aber mit beiden gleichzeitig. Diese Öffnung, die in einem Bodenbereich oder unteren Bereich (unteres Drittel, z. B.) des Effluentbeutels vorgesehen sein kann, wird somit sowohl als Eingang als auch als Ausgang verwendet.

Vorteilhafterweise kann die Bilanziereinrichtung des Effluentbeutels, beispielsweise eine Hängewaage, bei Verwendung der erfindungsgemäßen Erfindung nicht beeinflusst werden und eine Bilanzierung nicht verfälscht werden, insbesondere nicht bei der Verwendung von dünnen Schlauchquerschnitten.

Ein weiterer Vorteil bei der Verwendung von dünnen Schlauchquerschnitten kann darin bestehen, dass diese Querschnitte, aufgrund der Oberflächenspannung der Flüssigkeiten darin, stets mit Flüssigkeit gefüllt sind. Somit gibt es kein Problem beim Entlüften des Schlauchs. Ein unkontrolliertes Nachfließen von Flüssigkeit, die ein vorhandenes Waagensystem oder eine vorhandene Wiegevorrichtung negativ beeinflussen könnte, wird vorteilhaft verhindert.

Da jeweils der Anwender erfindungsgemäß gezwungen wird, die Behandlung zu unterbrechen und die Effluentzulaufleitung von der Effluentöffnung getrennt haben muss, bevor diese mit der Effluentablaufleitung verbunden werden kann und in umgekehrter Reihenfolge vorgehen muss, um die Blutbehandlung eines Patienten fortzusetzen, ist das Set automatisch vor Fehlbenutzung geschützt, was auch der Patientensicherheit zugutekommen kann.

Die Flusswege zwischen Ausguss oder Abfluss einerseits und Dialysator oder Patient andererseits sind in der vorliegenden Erfindung somit vorteilhafterweise elektrisch voneinander getrennt, was der Sicherheit des Patienten dient.

Ein weiterer Vorteil der vorliegenden Erfindung besteht im geringen Aufwand, der zur Implementierung der Erfindung erforderlich ist.

Bekannte Effluentbeutel, die über nur eine Öffnung verfügen, welche sowohl zum Befüllen als auch zum Entleeren des Effluentbeutels verwendet werden, benötigen eine Umschalteinrichtung wie z. B. einen Drei-Wege-Hahn, mittels welcher zwischen einer Stellung zum Fördern zum Befüllen und einer Stellung zum Fördern zum Entleeren umgeschaltet werden kann. Um elektrische Ströme zu vermeiden, müssen bei solchen Lösungen weitere Vorkehrungen getroffen werden. Dies ist im Falle der vorliegenden Erfindung nicht erforderlich. Sie stellt sicher, dass entweder nur die Blutbehandlungsvorrichtung oder nur der Ausguss mit dem Effluentbeutel in Verbindung stehen kann, ohne dass es einen nennenswert materiellen Aufwand bedeuten würde. Weitere Komponenten als die hierin genannten sind für das erfindungsgemäße Set nicht erforderlich.

Durchmesserunterschiede, welche zwischen bekannten Disposable-Bestandteilen im Bereich des Effluentbeutels vorliegen, werden erfindungsgemäß mittels passender Konnektoren und/oder angepasster Durchmesser überwunden.

Der Adapter des Sets erlaubt es ferner bekannte Effluentbeutel mit nur einer Effluentöffnung auch an Blutbehandlungsvorrichtungen zu verwenden, welche für eine Verwendung gemeinsam mit solchen Effluentbeuteln nicht vorgesehen waren, weshalb dies bislang nicht möglich war.

Das erfindungsgemäße Set schlägt mit seinem Adapter eine technische Lösung vor, die sich an bestehende Lösungen anschließen lässt, ohne Letztere zu verändern. Ein erneutes Zulassen z. B. der Effluentzulaufleitung, wenn diese z. B. in anderer Ausführung oder Länge als bisher verwendet werden sollte, oder der hiermit verbundenen Blutbehandlungsvorrichtung als medizinisches Gerät ist somit vorteilhafter Weise nicht erforderlich, was Mühe, Kosten und Aufwand spart.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt ein erfindungsgemäßes Set in einer ersten Ausführungsform;
- **Fig. 2**: zeigt das erfindungsgemäße Set der Fig. 1, wobei der Adapter mittels seines zweiten Konnektors vom ersten Typ mit dem ersten Konnektor vom ersten Typ aus Fig. 1 verbunden ist;
- **Fig. 3**: zeigt wiederum das erfindungsgemäße Set der Fig. 1, wobei der Adapter des Sets nicht mehr in Fluidverbindung mit dem Effluentbeutel der Fig. 2 verbunden ist;
- **Fig. 4**: zeigt in stark vereinfachter Darstellung einen Teil eines erfindungsgemäßen Sets mit Leitungs-, Schlauchabschnitten und Konnektoren;
- **Fig. 5**: zeigt in stark vereinfachter Darstellung eine Blutbehandlungsvorrichtung eines erfindungsgemäßen Sets, verbunden mit einem extrakorporalen Blutkreislauf und mit einem Effluentbeutel des erfindungsgemäßen Sets; und
- **Fig. 6**: zeigt in vereinfachter Darstellung ein erfindungsgemäßes Set mit einem erfindungsgemäßen Effluentbeutel, während dem Effluentbeutel Effluent zugeführt wird; und
- **Fig. 7**: zeigt das Set der Fig. 6, während aus dem Effluentbeutel Effluent abgeführt wird.

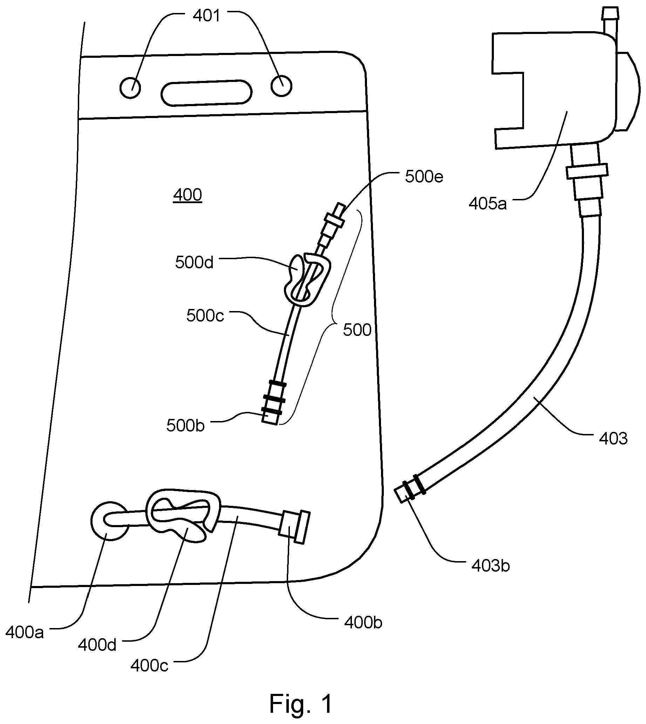

**Fig. 1** zeigt ein erfindungsgemäßes Set, umfassend oder bestehend aus einen Effluentbeutel 400 und einem Adapter 500.

Der Effluentbeutel 400 dient im Gebrauch zum Aufnehmen des bei einer Blutbehandlung anfallenden Effluents. Er weist eine verschließbare Effluentöffnung 400a auf, die der Verbindung des Inneren des Effluentbeutels 400 mit dessen Äußeren dient.

Zum Verbinden der Effluentöffnung 400a weist diese einen ersten Konnektor 400b von einem ersten Typ auf oder ist hiermit verbunden.

Zum Verschließen der Effluentöffnung 400a kann die hier gezeigte, rein optional vorgesehene Klemme 400d dienen. Sie ist hier an einem ebenfalls rein optional vorgesehenen Schlauchabschnitt 400c vorgesehen, welcher die Effluentöffnung 400a mit dem ersten Konnektor 400b vom ersten Typ verbindet. Wäre kein solcher Schlauchabschnitt 400c vorgesehen, so könnte der erste Konnektor 400b vom ersten Typ unmittelbar mit der Effluentöffnung 400a verbunden sein.

Der Adapter 500 weist einen Leitungsabschnitt, hier als Schlauchabschnitt 500c ausgestaltet, mit zwei Öffnungen auf, durch welche Fluid im Gebrauch des Adapters 500 in diesen hinein bzw. aus diesem heraus gefördert wird, und welche mittels des Schlauchabschnitts 500c miteinander verbunden sind. Das Lumen des Schlauchabschnitts 500c ist mittels einer optionalen Klemme 500d des Adapters 500 verschließbar.

Jede der vorgenannten beiden Öffnungen weist jeweils einen Konnektor auf, konkret einen zweiten Konnektor 500b vom ersten Typ und einen dritten Konnektor 500e vom zweiten Typ.

Der zweite Konnektor 500b ist, wie erwähnt, wie der erste Konnektor 400b vom ersten Typ. Dabei kann es sich zwar um ein weibliches und ein männliches Gegenstück handeln, weshalb der erste Konnektor 400b vom ersten Typ und der zweite Konnektor 500b vom ersten Typ nicht identisch ausgestaltet sind. Sie können aber dennoch miteinander verbunden werden, daher gelten sie hier als Konnektoren vom selben Typ. Ein Konnektor vom ersten Typ wäre hingegen nicht mit einem Konnektor eines zweiten Typs verbindbar.

Der dritte Konnektor 500e ist ein solcher Konnektor vom zweiten Typ.

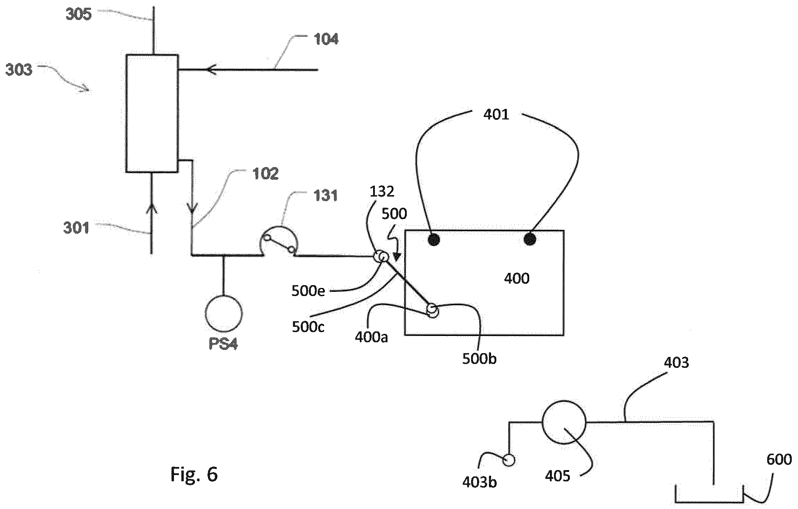

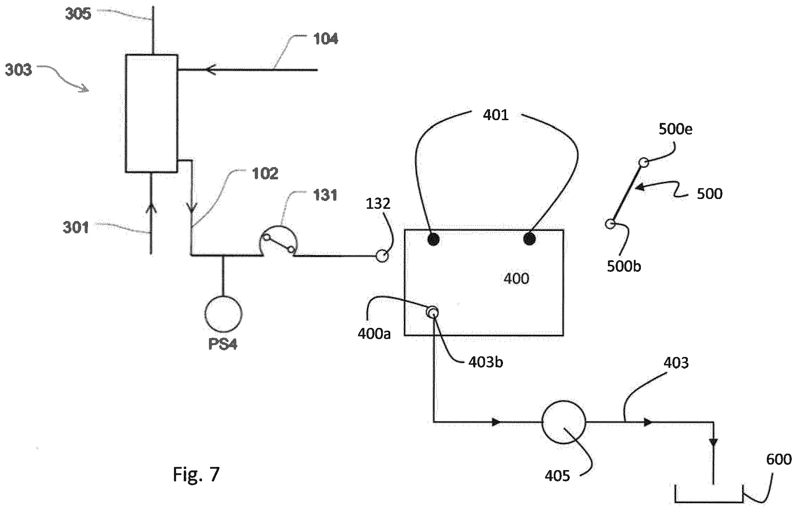

Rechts in Fig. 1 ist eine Effluentablaufleitung 403, im Gebrauch verbunden mit einem Pumpenkopf 405a einer in Fig. 6 und Fig. 7 gezeigten Pumpe 405, welche dem Abpumpen von Effluent aus dem Effluentbeutel 400 dient.

Die Effluentablaufleitung 403 weist einen vierten Konnektor 403b vom ersten Typ auf.

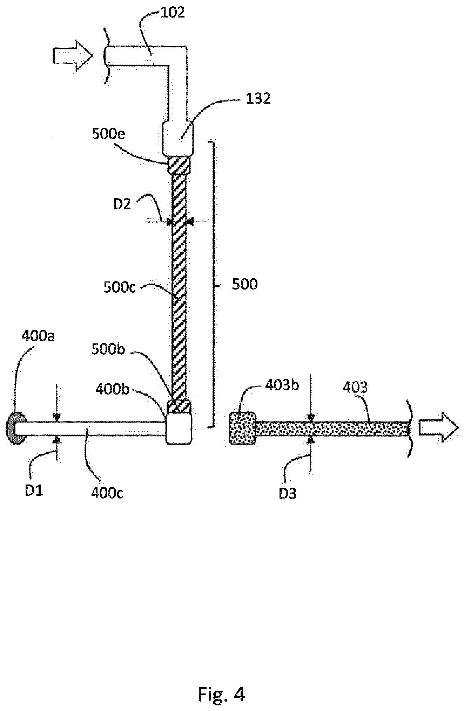

Von den in Fig. 1 gezeigten Konnektoren lassen sich bestimmungsgemäß somit folgende Paarungen fluiddicht verbinden: erster Konnektor 400b mit zweitem Konnektor 500b, beide vom ersten Typ; erster Konnektor 400b mit dem vierten Konnektor 403b, beide vom ersten Typ (wie zu Fig. 4 näher beschrieben).

Der Effluentbeutel 400 kann Befestigungsöffnungen 401 aufweisen, mittels welcher er z. B. an Haken einer Wiegevorrichtung der Blutbehandlungsvorrichtung 100 aufgehängt werden kann.

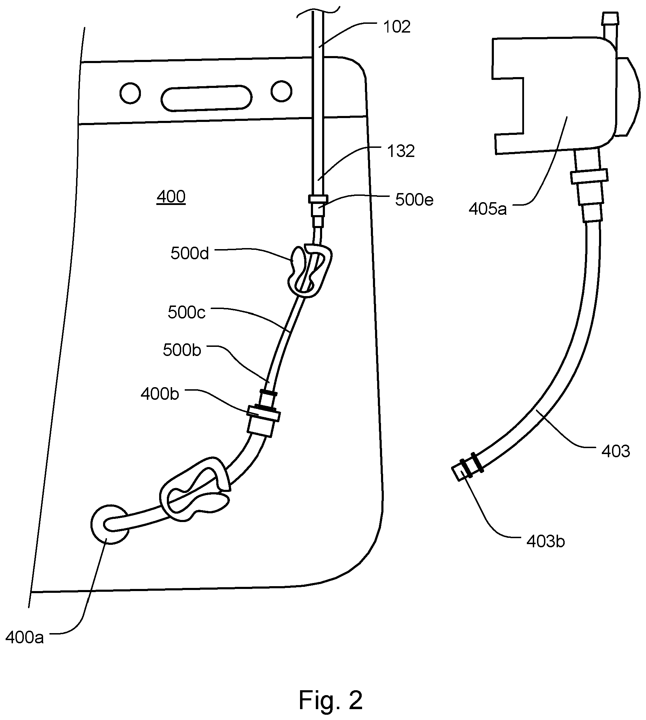

**Fig. 2** zeigt das erfindungsgemäße Set der Fig. 1, wobei der Adapter 500 mittels seines zweiten Konnektors 500b vom ersten Typ mit dem ersten Konnektor 400b vom ersten Typ verbunden ist.

Der dritte Konnektor 500e vom zweiten Typ ist bestimmungsgemäß am in der in Fig. 2 angedeuteten Effluentzulaufleitung 102 vorgesehenen fünften Konnektor 132 vom zweiten Typ zum Befüllen des Effluentbeutels 400 verbunden.

Der in Fig. 2 gezeigte Verbindungszustand dient dem Befüllen des Effluentbeutels 400.

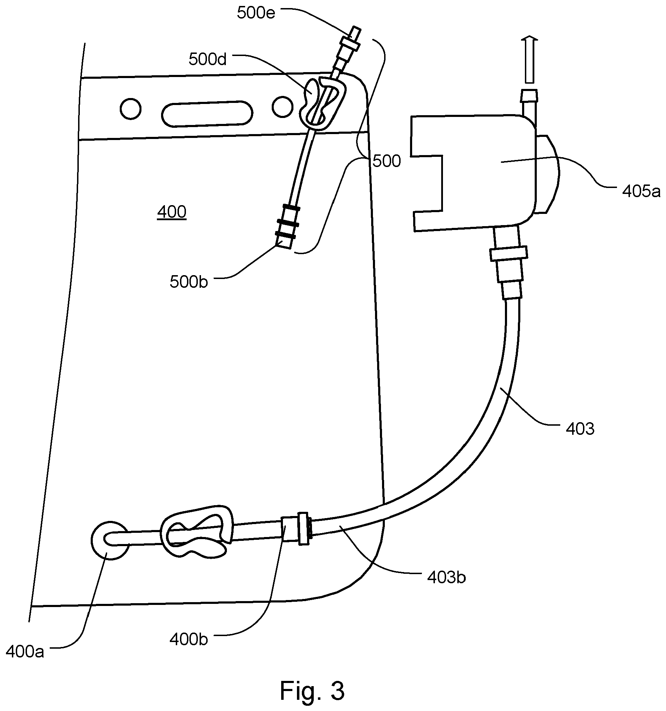

**Fig. 3** zeigt wiederum das erfindungsgemäße Set der Fig. 1, wobei der Adapter 500 nicht mehr in Fluidverbindung mit dem Effluentbeutel 400 verbunden ist.

Vielmehr ist in Fig. 3 ein Verbindungszustand gezeigt, mittels welchem der Effluentbeutel 400 über die Effluentablaufleitung 403, welche ihrerseits, direkt oder indirekt, mit dem Ausguss 600 verbunden sein kann, entleert werden könnte. Hierzu sind der erste Konnektor 400b vom ersten Typ und der vierte Konnektor 403b vom ersten Typ miteinander verbunden.

Man sieht in der Zusammenschau der Fig. 2 und 3, dass zur gleichen Zeit stets nur eine Verbindungsmöglichkeit gegeben ist, entweder die der Fig. 2 oder die der Fig. 3. Damit ist beim Entleeren des Effluentbeutels 400 dieser gänzlich fluidisch von der Behandlungsvorrichtung 100 bzw. deren Effluentzulaufleitung 102 bzw. deren Konnektor, hierin als fünfter Konnektor 132 vom zweiten Typ bezeichnet, getrennt.

**Fig. 4** zeigt in stark vereinfachter Darstellung einen Teil eines Sets mit Leitungsabschnitten 102, 403, Schlauchabschnitten 400c, 500c, und Konnektoren 132, 500e, 400b, 500b, 403b. Dabei sind die Konnektoren 400b, 500b, 403b vom ersten Typ, die Konnektoren 132 und 500e vom zweiten Typ. Der Adapter 500 des erfindungsgemäßen Sets ist schraffiert dargestellt. Über die Effluentzulaufleitung 102 und den fünften Konnektor 132 vom zweiten Typ wird bei der Blutbehandlung mit der Blutbehandlungsvorrichtung 100 angefallenes Effluent in den Adapter 500 gefördert. Dies ist mit dem linken Pfeil dargestellt. Die Effluentablaufleitung 403 mit dem vierten Konnektor 403b vom ersten Typ ist gepunktet gezeigt. Wenn diese mit dem Effluentbeutel 400 verbunden ist, wird das Effluent in einen Ausguss 600 gefördert. Dies ist mit dem rechten Pfeil veranschaulicht.

Von den in Fig. 4 gezeigten Konnektoren lassen sich bestimmungsgemäß die folgenden Paarungen, insbesondere fluiddicht, miteinander verbinden:
- erster Konnektor 400b mit zweitem Konnektor 500b, beide vom ersten Typ (wie dargestellt);
- dritter Konnektor 500e mit fünftem Konnektor 132, beide vom zweiten Typ (wie dargestellt); ferner
- erster Konnektor 400b mit viertem Konnektor 403b, beide vom ersten Typ (hier nicht dargestellt, siehe aber Fig. 3 und Fig. 7).

Die vorgenannten Konnektoren lassen sich beispielsweise miteinander verbinden, da sie beispielsweise dieselbe Form oder komplementäre Formen und/oder Größe aufweisen.

Die vorgenannten Konnektoren lassen sich beispielsweise miteinander verbinden, da sie bestimmungsgemäß zu einem Verbinden miteinander vorgesehen sind, da sie vom selben Typ (optional aber dennoch als männliches und weibliches Pendant ausgestaltet) sind.

Die vorgenannten Konnektoren lassen sich beispielsweise miteinander verbinden ohne Werkzeuge, Zusatzmaterial, usw. einsetzen zu müssen.

Konnektoren, die sich nicht bestimmungsgemäß, insbesondere fluiddicht, miteinander verbinden lassen, lassen ihre versehentliche Verbindung miteinander nicht zu.

Der dritte Konnektor 500e vom zweiten Typ lässt sich mit keinem der Konnektoren 400b, 500b und 403b vom ersten Typ fluiddicht verbinden.

Der Innendurchmesser des Schlauchabschnitts 400c, hierin als erster Innendurchmesser D1 bezeichnet, ist größer als der zweite Innendurchmesser D2 des Schlauchabschnitts 500c, hierin als D2 bezeichnet.

Der Innendurchmesser D2 des Schlauchabschnitts 500c ist kleiner als der Innendurchmesser D3 der Effluentablaufleitung 403.

Der Innendurchmesser D1 des Schlauchabschnitts 400c und der Innendurchmesser D3 der Effluentablaufleitung 403 können in einigen Ausführungsformen gleich sein.

Der Innendurchmesser der Effluentzulaufleitung 102 und der Innendurchmesser D2 des Adapters 500 können in einigen Ausführungsformen gleich sein.

Rein exemplarisch ist der dritte Konnektor 500e vom zweiten Typ männlich, z. B. ein männlicher Luer-Lock-Verbinder. Auch der zweite Konnektor 500b vom ersten Typ ist rein exemplarisch männlich, z. B. ein männlicher Luer-Lock-Verbinder. Dasselbe gilt optional für den vierten Konnektor 403b vom ersten Typ. Er kann - wie andere Konnektoren hierin ebenfalls - eine Luer-Lock-Verbindung oder von einer anderen Bauart sein, z. B. ein COLDER-Verbinder, d. h. eine Ausführungsform einer Schnellkupplung, ausgeführt als Steckverbinder mit radialer Dichtung und federbelasteter Verriegelung.

Der erste Konnektor 400b vom ersten Typ ist hingegen vorzugsweise weiblich, z. B. ein weiblicher Luer-Lock-Verbinder.

Der erste bzw. der dritte Innendurchmesser D1 bzw. D3 kann in manchen Ausführungsformen zwischen 5 mm und 15 mm, vorzugsweise 10 mm, betragen.

Der zweite Innendurchmesser kann zwischen 3 mm und 7 mm betragen, vorzugsweise 4,3 mm.

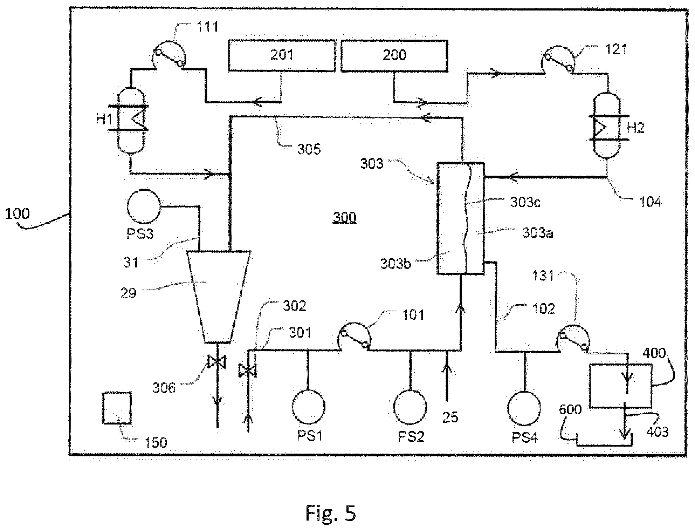

**Fig. 5** zeigt in stark vereinfachter Darstellung eine Blutbehandlungsvorrichtung 100 eines erfindungsgemäßen Sets, verbunden mit einem extrakorporalen Blutkreislauf 300 und einem Effluentbeutel 400 des erfindungsgemäßen Sets.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zu ihrer Verbindung mit dem Gefäßsystem des nicht dargestellten Patienten dienen.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 1 nur durch einige ihrer Einrichtungen und schematisch repräsentierte Blutbehandlungsvorrichtung 100 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200 entlang der Dialysierflüssigkeitszulaufleitung 104 in die Dialysierflüssigkeitskammer 303a gepumpt. Die Dialysierflüssigkeit verlässt die Dialysierflüssigkeitskammer 303a als Dialysat, ggf. angereichert durch Filtrat, in Richtung des Ausgusses 600 und wird hierin als Effluent bezeichnet.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Eine nur angedeutete Steuer- oder Regelvorrichtung 150 kann dazu konfiguriert sein, das vorstehende Verfahren auszuführen. Optional erfolgt eine manuelle Ausführung.

Rechts unten ist in Fig. 5 angedeutet, wo die Effluentablaufleitung 403 mit dem in Fig. 1 gezeigten Effluentbeutel 400 optional mit der Blutbehandlungsvorrichtung 100 verbunden ist.

Neben der vorgenannten Blutpumpe 101 weist die in Fig. 5 gezeigte Anordnung ferner rein optional eine Reihe weiterer, jeweils optionaler Pumpen, nämlich die Pumpe 111 für Substituat, die Pumpe 121 für Dialysierflüssigkeit und die Pumpe 131 für das Effluent auf.

Die Pumpe 121 ist vorgesehen, um Dialysierflüssigkeit aus einer Quelle 200, beispielsweise einem Beutel, heraus und über eine optional vorhandene Beutelheizung H2 mit einem Heizbeutel dem Blutfilter 303 mittels einer Dialysierflüssigkeitszulaufleitung 104 zuzuführen.

Die auf diese Weise zugeführte Dialysierflüssigkeit tritt über eine Dialysatablaufleitung 102, unterstützt durch die Pumpe 131, wieder aus dem Blutfilter 303 und kann verworfen werden.

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, stromauf einer Zugabestelle 25 für Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 aufweisen und mit einem weiteren Drucksensor PS3 in Fluidverbindung stehen kann.

Die in Fig. 5 gezeigte exemplarische Anordnung weist eine Steuer- oder Regelvorrichtung 150 auf. Sie kann mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen. Sie ist optional konfiguriert, um das hierin beschriebene Verfahren auszuführen.

Die optionale Pumpe 111 ist vorgesehen, um Substituat aus der optionalen Quelle 201, beispielsweise einem Beutel, heraus und über eine optional vorhandene Beutelheizung H1 mit einem Heizbeutel der zweiten Leitung 305 zuzuführen.

**Fig. 6** zeigt in vereinfachter Darstellung ein erfindungsgemäßes Set mit dessen Effluentbeutel 400 in einem Moment, während welchem dem Effluentbeutel 400 Effluent zugeführt wird.

Die Effluentablaufleitung 403 kann eine optionale Pumpe 405 aufweisen oder hiermit verbunden sein und schließlich in den Ausguss 600 münden.

Die Pumpe 405 ist stromab des Effluentbeutels 400, aber stromauf des Ausgusses 600, angeordnet.

Die Pumpe 405, welche, wie erst in Fig. 4 gezeigt, optional wenigstens einen Pumpenantrieb und einen Pumpkopf 405a aufweist oder hieraus bestehen kann, ist in der Stellung der Fig. 6 nicht in Betrieb ("OFF"), da die Effluentablaufleitung 403 kein Effluent führt, welche mittels der Pumpe 405 in den Ausguss 600 hinein verworfen werden könnte.

Die Pumpe 405 kann in jeder Ausführungsform eine Rollenpumpe sein. Alternativ ist sie in beliebigen Ausführungsformen keine Rollenpumpe, sondern z. b. eine Impellerpumpe oder eine Membranpumpe.

Die Effluentablaufleitung 403 ist mit dem Pumpkopf 405a der Pumpe 405 fluidisch verbunden. Der Pumpkopf 405a kann Teil der Effluentablaufleitung 403 sein, die wiederum ein Einweg-Schlauch sein kann. Der Pumpkopf 405a kann magnetisch gelagert sein, was sein Verbinden mit weiteren Abschnitten der Pumpe 405 vereinfachen kann und eine Übertragung von Strom zwischen den verbundenen Bauteilen vorteilhaft zu vermeiden hilft.

Die Druckseite des Pumpkopfs ist mit einer Leitung verbunden, die als eine Fortsetzung der Effluentablaufleitung 403 oder aber als weiterer Schlauchabschnitt verstanden werden kann.

Der Pumpenantrieb der Pumpe 405, welcher mit dem Pumpkopf zusammenwirkt, um die Pumpe 405 zu bilden, kann Teil der Blutbehandlungsvorrichtung 100 sein. Optional ist er hingegen Teil einer, z. B. mobilen, Vorrichtung. Letztere hat vorzugsweise keinen elektrischen und/oder körperlichen Kontakt mit der Blutbehandlungsvorrichtung. Vorzugsweise wird die Vorrichtung von einer, vorzugsweise ladbaren, Spannungsquelle gespeist, die beim Betrieb der Pumpe nicht mit dem Stromnetz der Blutbehandlungsvorrichtung und/oder der Klinik verbunden ist.

Der Effluentbeutel 400 ist vorzugsweise mit einer Wiegeeinrichtung zum Wiegen seines Gewichts oder des in ihm aufgenommenen Fluids oder zum Bestimmen einer Gewichtsänderung verbunden. Beispielsweise kann der Effluentbeutel 400 als Auffangbeutel auf einer Wiegefläche der Wiegeeinrichtung liegen oder mit seinen Befestigungsöffnungen 401 an einem oder mehreren nicht gezeigten Wiegehaken der Blutbehandlungsvorrichtung 100 hängen.

Wie Fig. 6 zu entnehmen ist, weist das Set optional eine Pumpe 405 auf. Das Effluent entleert sich in alternativen Ausführungsformen ohne Pumpenunterstützung, z. B. allein mittels Schwerkraft, aus dem Effluentbeutel 400 heraus.

Ein nicht gezeigtes Rückschlagventil kann in der Effluentzulaufleitung 102 vorgesehen sein.

In der in Fig. 6 gezeigten Stellung des Sets und insbesondere seines Adapters 500 kommt dieser zum Einsatz, und zwar zum Befüllen des Effluentbeutels 400.

Der zweite Konnektor 500b vom ersten Typ des Adapters 500 ist mit dem ersten Konnektor 400b vom ersten Typ des Effluentbeutels 400 verbunden.

Der dritte Konnektor 500e vom zweiten Typ des Adapters 500 ist mit dem fünften Konnektor 132 vom zweiten Typ der Effluentzulaufleitung 102 verbunden.

Der Effluentspiegel im Inneren des Effluentbeutels 400 steigt in dieser Phase an.

Flüssigkeitsströme sind mittels Pfeilen angedeutet.

**Fig. 7** zeigt das Set der Fig. 6, während aus dem Effluentbeutel 400 Effluent abgeführt wird.

Zum Abpumpen des Effluentbeutels 400 ist die Pumpe 131 für das Effluent angehalten. Dadurch wird der stromaufwärts der Pumpe 131 verlaufende, flüssigkeitsgefüllte Abschnitt der Effluentzulaufleitung 102 von dem stromabwärts der Pumpe 131 verlaufenden Abschnitt der flüssigkeitsgefüllten Effluentzulaufleitung 102 elektrisch isoliert. Hierdurch werden die zulässigen Grenzen der Patientenableitströme unterschritten. Die stehende, okkludierende Pumpe 131 (Rollenpumpe) isoliert dabei die stromauf der Pumpe 131 bzw. stromab der Pumpe 131 vorliegenden Flüssigkeitssäulen voneinander. Die herrschende Schwerkraft mag ein Übriges hierzu beitragen, indem die stromab der Pumpe 131 vorliegende Flüssigkeitssäule schwerkraftbedingt in Richtung des Effluentbeutels 400 abreißt oder sich in dessen Richtung bewegt.

Zum Entleeren des Effluentbeutels 400 wird die Effluentzulaufleitung 102 vom Effluentbeutel 400 getrennt. Dies kann mittels des fünften Konnektors 132 vom zweiten Typ, der den stromabwärts der Pumpe 131 verlaufenden Abschnitt der flüssigkeitsgefüllten Effluentzulaufleitung 102 mit dem Effluentbeutel 400 in Fig. 6 noch verbunden hat, erfolgen. Die Effluentzulaufleitung 102 kann mittels einer in Fig. 6 nicht dargestellten Klemme ferner abgeklemmt werden.

Die Effluentzulaufleitung 102 kann nach ihrer Trennung optional mit einer Kappe verschlossen werden und/oder mittels einer manuellen Schlauchklemme (Kappe und Schlauchklemme sind in Fig. 7 nicht gezeigt) stromabwärts der Pumpe 131 geschlossen werden.

Insbesondere dann, wenn die Pumpe 405 eine Impellerpumpe ist (sie ist nicht hierauf beschränkt), die nicht selbst ansaugend ist, kann es von Vorteil sein, wenn die Effluentablaufleitung 403 in Richtung Ausguss 600 ansteigend verlegt wird. Alternativ oder optional verläuft die Effluentablaufleitung 403 zwischen einem nicht gezeigten Absperrelement und der Pumpe 405 abfallend verlegt, sodass Luftblasen aufsteigen und in Richtung Ausguss 600 oder alternativ Richtung Effluentbeutel 400 abgeführt werden können.

Bei diesem mit Bezug auf Fig. 7 erläuterten Verfahren ist mit dem Ziel der elektrischen Sicherheit (d. h. zum sicheren Unterschreiten der Grenzwerte für die Patientenableitströme auch im Fehlerfall) vorteilhafterweise ein Entleeren des Effluentbeutels 400 erst möglich, wenn die Effluentzulaufleitung 102 vom Effluentbeutel 400 getrennt wurde.

Die Pumpe 405 kann als Pumpe, ausgestaltet sein, wie sie in der Deutschen Patentanmeldung, die für die Anmelderin der vorliegenden Anmeldung unter dem Aktenzeichen DE 102017122804.7 mit Anmeldetag 29. September 2017 beim Deutschen Patent- und Markenamt angemeldet wurde, offenbart ist. Deren diesbezügliche Offenbarung wird hiermit durch Verweis zum Gegenstand auch der vorliegenden Anmeldung gemacht. Es kann aber alternativ eine andere Pumpe verwendet werden.

Optional weist das Set stromab des Effluentbeutels 400 keinen Flussteiler auf. Das Set kann hingegen eine Rollenpumpe aufweisen, etwa als Pumpe 405. Die Rollenpumpe kann optional mit genau einer Zuleitung und mit genau einer Ableitung verbunden sein. Damit kann die Pumpe den in sie hineinführenden Fluss offensichtlich nicht in mehrere Flüsse teilen.

Optional weist das Set stromab der Pumpe 405 keinen Konnektor auf.

Optional weist das Set, das sich stromab des Effluentbeutels 400 und/oder stromauf der Pumpe 405 anschließt, kein Element auf, das mit einer elektrischen Steuereinrichtung verbunden wäre, etwa in Form eines elektrisch verbundenen Verbinders.

### Bezugszeichenliste

- 25: Zugabestelle für Heparin (optional)
- 29: venöse Blutkammer (optional)
- 31: Entlüftungseinrichtung

- 100: Blutbehandlungsvorrichtung
- 101: Blutpumpe
- 102: Dialysatablaufleitung, Effluentzulaufleitung
- 104: Dialysierflüssigkeitszulaufleitung
- 111: Pumpe für Substituat
- 121: Pumpe für Dialysierflüssigkeit
- 131: Pumpe für Dialysat oder Effluent in Effluentzulaufleitung
- 132: fünfter Konnektor vom zweiten Typ

- 150: Steuer- oder Regelvorrichtung

- 200: Quelle mit Dialysierflüssigkeit
- 201: Quelle mit Substituat, optional

- 300: extrakorporaler Blutkreislauf
- 301: erste Leitung (arterieller Leitungsabschnitt)
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: (zweite) Schlauchklemme

- 400: Effluentbeutel
- 400a: Effluenteinlass- oder -auslassöffnung; Effluentöffnung
- 400b: erster Konnektor vom ersten Typ
- 400c: Schlauchabschnitt
- 400d: Klemme
- 401: Befestigungsöffnungen

- 403: Effluentablaufleitung
- 403b: vierter Konnektor vom ersten Typ

- 405: Pumpe in Effluentablaufleitung
- 405a: Pumpkopf

- 500: Adapter
- 500b: zweiter Konnektor vom ersten Typ
- 500c: Schlauchabschnitt
- 500d: Klemme
- 500e: dritter Konnektor vom zweiten Typ

- 600: Ausguss

- D1, D2, D3: Innendurchmesser
- H2: Beutelheizung mit Beutel (Dialysierflüssigkeit)
- H1: Beutelheizung mit Beutel (Substituat)

- PS1, PS2: arterieller Drucksensor (optional)
- PS3: Drucksensor (optional)
- PS4: Drucksensor zum Messen des Filtratdrucks

## Patentansprüche

1. Set, umfassend oder bestehend aus:
- einem Effluentbeutel (400) zum Aufnehmen des bei einer Blutbehandlung anfallenden Effluents, mit genau einer verschließbaren Effluentöffnung (400a) zu einem Äußeren des Effluentbeutels (400), wobei die Effluentöffnung (400a) einen ersten Konnektor (400b) von einem ersten Typ aufweist oder hiermit verbunden ist;
- einem Adapter (500) mit einem Leitungsabschnitt zum Leiten eines Fluids, wobei der Leitungsabschnitt einen zweiten Konnektor (500b) vom ersten Typ und einen dritten Konnektor (500e) von einem zweiten, vom ersten Typ verschiedenen Typ aufweist, wobei der dritte Konnektor (500e) konfiguriert ist, um mit einem Konnektor (132) vom zweiten Typ einer Effluentzulaufleitung (102) einer Blutbehandlungsvorrichtung (100) verbunden zu werden.

2. Set nach Anspruch 1, wobei
- der Leitungsabschnitt des Adapters (500) entweder eine nicht biegsame Kunststoffleitung oder ein Schlauchabschnitt (500c) ist oder jeweils solche aufweist.

3. Set nach einem der vorangegangenen Ansprüche, wobei
- die Effluentöffnung (400a) mit dem ersten Konnektor (400b) vom ersten Typ mittels eines Schlauchabschnitts (400c) in Fluidverbindung steht, welcher zwischen Effluentöffnung (400a) und erstem Konnektor (400b) vom ersten Typ einen ersten Innendurchmesser (D1) aufweist.

4. Set nach Anspruch 3, wobei
- der Leitungsabschnitt des Adapters (500) einen zweiten Innendurchmesser (D2) aufweist, welcher geringer ist als der erste Innendurchmesser (D1).

5. Set nach einem der vorangegangenen Ansprüche, wobei
- der Adapter (500) - insbesondere der als Schlauchabschnitt (500c) ausgestaltete Leitungsabschnitt des Adapters (500) - mit einer äußeren Oberfläche des Effluentbeutels (400), vorzugsweise lösbar, verbunden ist.

6. Set nach einem der vorangegangenen Ansprüche, weiter aufweisend
- eine Effluentablaufleitung (403) zum Abführen von Effluent aus dem Effluentbeutel (400), wobei die Effluentablaufleitung (403) einen vierten Konnektor (403b) von einem ersten Typ und/oder einen Innendurchmesser aufweist, welcher dem ersten Innendurchmesser (D1) im Wesentlichen oder genau entspricht.

7. Set nach einem der vorangegangenen Ansprüche, weiter aufweisend
- wenigstens eine Pumpe (405) oder einen Pumpkopf (405a) einer Pumpe (405), verbunden mit der Effluentablaufleitung (403) zum Pumpen von Effluent aus dem Effluentbeutel (400) heraus, oder zu ihrer Verbindung mit der Effluentablaufleitung (403) vorgesehen.

8. Set nach einem der vorangegangenen Ansprüche, weiter aufweisend
- eine Blutbehandlungsvorrichtung (100) mit einer Effluentzulaufleitung (102) zum Zuführen von bei einer Blutbehandlungssitzung angefallenen Effluents in den Effluentbeutel (400) hinein, wobei die Effluentzulaufleitung (102) einen fünften Konnektor (132) vom zweiten Typ aufweist.

9. Set nach einem der vorangegangenen Ansprüche, wobei die Blutbehandlungsvorrichtung (100) ausgestaltet ist als Hämodialysevorrichtung, Hämofiltrationsvorrichtung oder Hämodiafiltrationsvorrichtung, insbesondere als eine Vorrichtung für die chronische Nierenersatztherapie oder für die kontinuierliche Nierenersatztherapie (CRRT = *continuous renal replacement therapy*)*.*

10. Verfahren zum Entleeren eines Effluentbeutels (400), mit den Schritten:
- Bereitstellen eines Sets gemäß einem der Ansprüche 8 bis 9, wobei der erste Konnektor (400b), vom ersten Typ, des Effluentbeutels (400) zum Aufnehmen des bei einer Blutbehandlung angefallenen Effluents im Effluentbeutel (400) mit dem zweiten Konnektor (500b), vom ersten Typ, des Adapters (500) verbunden ist; und wobei der dritte Konnektor (500e), vom zweiten Typ, des Adapters (500) mit dem fünften Konnektor (132), vom zweiten Typ, der Effluentzulaufleitung (102) der Blutbehandlungsvorrichtung (100) verbunden ist;
- Trennen des ersten Konnektors (400b) vom ersten Typ vom zweiten Konnektor (500b) vom ersten Typ;
- Verbinden des ersten Konnektors (400b), vom ersten Typ, mit dem vierten Konnektor (403b), vom ersten Typ, der Effluentablaufleitung (403) zum Abführen von Effluent aus dem Effluentbeutel (400) derart, dass eine Fluidverbindung zwischen dem Inneren des Effluentbeutels (400) und dem Inneren der Effluentablaufleitung (403) hergestellt wird.

11. Verfahren nach Anspruch 10, mit dem Schritt
- Fördern von Effluent aus dem Effluentbeutel (400) heraus, nachdem der erste Konnektor (400b), vom ersten Typ, des Effluentbeutels (400) mit dem vierten Konnektor (403b), vom ersten Typ, der Effluentablaufleitung (403) verbunden wurde.

## Claims

1. A set comprising or consisting of:
- an effluent bag (400) for collecting an effluent generated during a blood treatment, with exactly one closable effluent opening (400a) to an exterior of the effluent bag (400), wherein the effluent opening (400a) comprises or is connected to a first connector (400b) of a first type;
- an adapter (500) having a line section for conducting a fluid, wherein the line section comprises a second connector (500b) of the first type and a third connector (500e) of a second type different from the first type, the third connector (500e) being configured to be connected to a connector (132) of the second type of an effluent inlet line (102) of a blood treatment apparatus (100).

2. The set according to claim 1, wherein
- the line section of the adapter (500) is or comprises respectively either a non-flexible plastic line or a hose section (500c).

3. The set according to any one of the preceding claims, wherein
- the effluent opening (400a) is in fluid communication with the first connector (400b) of the first type by means of a hose section (400c), which comprises a first inner diameter (D1) between the effluent opening (400a) and the first connector (400b) of the first type.

4. The set according claim 3, wherein
- the line section of the adapter (500) comprises a second inner diameter (D2), which is smaller than the first inner diameter (D1).

5. The set according to any one of the preceding claims, wherein
- the adapter (500) - in particular the line section of the adapter (500) designed as a hose section (500c) - is connected, preferably detachably, to an outer surface of the effluent bag (400).

6. The set according to any one of the preceding claims, further comprising:
- an effluent outlet line (403) for discharging effluent from the effluent bag (400), wherein the effluent outlet line (403) comprises a fourth connector (403b) of a first type and/or an inner diameter, which substantially or exactly corresponds to the first inner diameter (D1).

7. The set according to any one of the preceding claims, further comprising:
- at least one pump (405) or one pump head (405a) of a pump (405), connected to the effluent outlet line (403) for pumping effluent out of the effluent bag (400), or provided for its connection to the effluent outlet line (403).

8. The set according to any one of the preceding claims, further comprising:
- a blood treatment apparatus (100) having an effluent inlet line (102) for feeding effluent generated during a blood treatment session into the effluent bag (400), wherein the effluent inlet line (102) comprises a fifth connector (132) of the second type.

9. The set according to any one of the preceding claims, wherein the blood treatment apparatus (100) is designed as a hemodialysis apparatus, hemofiltration apparatus or hemodiafiltration apparatus, in particular as an apparatus for the chronic renal replacement therapy or for the continuous renal replacement therapy (CRRT = *continuous renal replacement therapy*)*.*

10. A method for emptying a effluent bag (400), comprising the steps of:
- providing a set according to any one of claims 8 to 9, wherein the first connector (400b), of the first type, of the effluent bag (400) for receiving effluent generated during a blood treatment in the effluent bag (400) is connected to the second connector (500b), of the first type, of the adapter (500); and wherein the third connector (500e), of the second type, of the adapter (500) is connected to the fifth connector (132), of the second type, of the effluent inlet line (102) of the blood treatment apparatus (100);
- disconnecting the first connector (400b) of the first type from the second connector (500b) of the first type;
- connecting the first connector (400b), of the first type, to the fourth connector (403b), of the first type, of the effluent outlet line (403) for discharging effluent from the effluent bag (400) such that a fluid connection is established between the interior of the effluent bag (400) and the interior of the effluent outlet line (403).

11. The method according to claim 10 comprising the step:
- conveying effluent out of the effluent bag (400), after the first connector (400b) of the first type, of the effluent bag (400) has been connected to the fourth connector (403b), of the first type, of the effluent outlet line (403).

## Revendications

1. Un kit comprenant ou étant composé de :
- une poche d'effluent (400) destinée à recueillir un effluent généré lors d'un traitement du sang, comprenant exactement une ouverture pour effluent refermable (400a) vers l'extérieur de la poche d'effluent (400),
où l'ouverture pour effluent (400a) comprend, ou est raccordée à, un premier connecteur (400b) d'un premier type;
- un adaptateur (500) comportant une partie de conduit destinée à acheminer un fluide, où la partie de conduit comprend un second connecteur (500b) du premier type ainsi qu'un troisième connecteur (500e) d'un second type différent du premier type, le troisième connecteur (500e) étant configuré pour être raccordé à un connecteur (132) du second type d'un conduit d'entrée d'effluent (102) d'un appareil de traitement du sang (100).

2. Le kit selon la première revendication, où
- la partie de conduit de l'adaptateur (500) est ou comprend respectivement soit un conduit en plastique non flexible, soit une section de tuyau (500c).

3. Le kit selon l'une quelconque des revendications précédentes, où
- l'ouverture pour effluent (400a) est en communication fluidique avec le premier connecteur (400b) du premier type via une section de tuyau (400c) qui comporte un premier diamètre intérieur (D1) entre l'ouverture pour effluent (400a) et le premier connecteur (400b) du premier type.

4. Le kit selon la revendication 3, où
- la partie de conduit de l'adaptateur (500) comporte un second diamètre intérieur (D2) qui est plus petit que le premier diamètre intérieur (D1).

5. Le kit selon l'une quelconque des revendications précédentes, où
- l'adaptateur (500) - notamment la partie de conduit de l'adaptateur (500) conçue comme une section de tuyau (500c) - est raccordé, de préférence de manière détachable, à une surface extérieure de la poche d'effluent (400).

6. Le kit selon l'une quelconque des revendications précédentes, comprenant en outre :
- un conduit d'évacuation d'effluent (403) destiné à évacuer l'effluent de la poche d'effluent (400), où le conduit d'évacuation d'effluent (403) comprend un quatrième connecteur (403b) d'un premier type et/ou d'un diamètre intérieur qui correspond sensiblement ou exactement au premier diamètre intérieur (D1).

7. Le kit selon l'une quelconque des revendications précédentes, comprenant en outre :
- au moins une pompe (405) ou une tête de pompe (405a) d'une pompe (405), raccordée au conduit d'évacuation d'effluent (403), destinée à pomper l'effluent hors de la poche d'effluent (400) ou prévue pour être raccordée au conduit d'évacuation d'effluent (403).

8. Le kit selon l'une quelconque des revendications précédentes, comprenant en outre :
- un appareil de traitement du sang (100) ayant un conduit d'entrée d'effluent (102) destiné à amener l'effluent généré pendant une séance de traitement du sang dans la poche d'effluent (400), où le conduit d'entrée d'effluent (102) comprend un cinquième connecteur (132) du second type.

9. Le kit selon l'une quelconque des revendications précédentes, où l'appareil de traitement du sang (100) est conçu comme un appareil d'hémodialyse, un appareil d'hémofiltration ou un appareil d'hémodiafiltration, notamment comme un appareil pour la thérapie de remplacement rénal chronique ou pour la thérapie de remplacement rénal continue (CRRT = *continuous renal replacement therapy*)*.*

10. Un procédé de vidange d'une poche d'effluent (400) comprenant les étapes suivantes :
- fournir un kit selon l'une des revendications 8 à 9, où le premier connecteur (400b), du premier type, de la poche d'effluent (400) destiné à recevoir l'effluent généré pendant un traitement du sang dans la poche d'effluent (400) est raccordé au second connecteur (500b), du premier type, de l'adaptateur (500) ; et où le troisième connecteur (500e), du second type, de l'adaptateur (500) est raccordé au cinquième connecteur (132), du second type, du conduit d'entrée d'effluent (102) de l'appareil de traitement du sang (100);
- séparer le premier connecteur (400b) du premier type du second connecteur (500b) du premier type;
- raccorder le premier connecteur (400b), du premier type, au quatrième connecteur (403b), du premier type, du conduit d'évacuation d'effluent (403) pour évacuer l'effluent de la poche d'effluent (400) de telle sorte qu'une communication fluidique soit établie entre l'intérieur de la poche d'effluent (400) et l'intérieur du conduit d'évacuation d'effluent (403).

11. Le procédé selon la revendication 10 comprenant l'étape suivante :
- acheminer l'effluent hors de la poche d'effluent (400), après que le premier connecteur (400b), du premier type, de la poche d'effluent (400) a été connecté au quatrième connecteur (403b), du premier type, du conduit d'évacuation d'effluent (403).
